Europäisches Patentamt

(19) European Patent Office

Office européen des brevets

(11) Publication number: **0 000 984**
**B1**

(12) **EUROPEAN PATENT SPECIFICATION**

(45) Date of publication of patent specification: **11.05.83**

(51) Int. Cl.³: **A 61 N 1/36**

(21) Application number: **78300237.1**

(22) Date of filing: **03.08.78**

(54) **Programmable pacer with variable amplifier gain.**

(30) Priority: **19.08.77 GB 3491777**
**19.06.78 US 917142**

(43) Date of publication of application:
**07.03.79 Bulletin 79/5**

(45) Publication of the grant of the patent:
**11.05.83 Bulletin 83/19**

(84) Designated Contracting States:
**DE FR GB NL SE**

(56) References cited:
**LU - A - 76 880**
**US - A - 3 830 242**

**ELECTRONICS, vol. 48, nr. 14, July 10, 1975, New York, C. J. HARTLEY: "Digital word sets gain of amplifier", pages 94, 95.**

(73) Proprietor: **BIOTRONIK Mess- und Therapiegeräte GmbH & Co Ingenieurbüro Berlin Sieversufer 8 D-1000 Berlin 47 (DE)**

(72) Inventor: **Keller, John Walter 8600 S.W. 54th Avenue Miami Florida (US)**

(74) Representative: **Christiansen, Henning, Dipl.-Ing. Unter den Eichen 108a D-1000 Berlin 45 (DE)**

Courier Press, Leamington Spa, England.

## Programmable pacer with variable amplifier gain

### Field of the invention

This invention relates to cardiac pacemakers.

### Background art

Pacemakers for generating artificial stimulating pulses for the heart, and which may be implanted in the body, are well known. Originally the electrical circuitry for such pacemakers was of analog design, but in recent years digital circuitry has been also employed. A digital approach to pacemakers has led to the evolution of programmable pacemakers—pacemakers having parameters such as pulse rates which are adjustable (programmable) once the pacemaker has been implanted. The programs can be changed from outside the patient's body by appropriate signal transmission to the implanted pacemaker and without surgery. Programmable pacemakers are described in for instance, British Specifications 1,385,954 and 1,398,875. Such pacemakers have circuitry to detect and decode signals transmitted outside the body and alter the program accordingly. In British Specification 1,385,954 the programming is accomplished by means of a magnetic field which is sensed by a magnetic reed switch; the opening and closing of the switch providing programming pulses to a program store. In British Specification 1,398,875 the programming is by means of radio frequency transmission and reception.

Many pacemakers are of the demand type—that is they only supply a stimulating pulse to the heart when a natural heart beat is absent. To accomplish this, demand pacemakers have means for sensing the presence or absence of natural heat beats and for actuating the stimulating pulse as appropriate.

Demand pacemakers normally have an input amplifier which receives the electrical signals detected at the heart, and the amplified signals are then employed to control the demand function of the pacemaker. Generally, signals provided by the input amplifier are employed to inhibit any artificial pulse being generated by the pacemaker: the absence of such signals being taken by the pacemaker as indicating the absence of a normal heart beat and causing artificial stimulating pulses to be generated.

Pacemakers are implanted in the body for a period of years, and during that time various parameters can change causing the effective signals supplied by the input amplifier to change in magnitude. Typical of such parameters is an increase in impedance of the body tissue as "seen" by the pacing electrode. But most prevalent is the wide range of electrode placement effectiveness which can result in not only very large but very small signals.

U.S. Patent 4,049,004 describes an implantable cardiac pacemaker with a number of externally programmable pacemaker functions including the sensitivity of the pacemaker response to signals at the heart. The pacemaker includes an R-wave input amplifier constructed as an operational amplifier whose output is employed for resetting the stimulating pulse generator, thereby giving the pacemaker a demand capability. The output of the operational amplifier is supplied to a field effect transistor whose switching threshold is varied from a master parameter control. The latter is a memory store whose contents may be varied after implantation from a remote source of programming signals. This arrangement has no effect on the overall amplification provided to the signal supplied to the R-wave amplifier and can cause erroneous control of the pacemaker in certain operational states. For example, with input signals of small amplitude (and which desirably require increased amplification) and with the field effect transistor on its least sensitive setting, the signal supplied to the transistor by the R-wave amplifier will be insufficient to switch it on and thus would not reset the pulse generator as required. Under some circumstances, therefore, the field effect transistor can cut off the signal which should be employed to reset the pulse generator.

### Disclosure of the invention

We have now devised a pacemaker circuit which enables the signal supplied by the input amplifier to be changed after implantation and which does enable, for example, input signals of small magnitude to be handled at increased amplification or without the risk of cutting off the signal to be employed for resetting the pulse generator. This has been accomplished by providing different levels of attenuation for the signal supplied to the input amplifier and by selecting the desired attenuation by means of a program held in a pacemaker memory store.

According to the invention there is provided an implantable cardiac pacemaker having a pacemaker function programmably alterable from a remote source of pacemaker function programming signals, comprising pulse generating means (1, 2, 3, 4,) for periodically issuing stimulating pulses, an input amplifier (12) for sensing signals at the heart and for resetting said generating means in response to a sensed signal, and memory means (18) responsive to signals from said remote source for varying the signal provided by the input amplifier for resetting said pulse generating means, characterised in that said varying means comprises a potential dividing network (11, 14) provided with an intermediate tap connected to the input of said input amplifier (12), means (13) connecting one extremity of said network to sense signals at the heart, and means (15, 16, 17) connected to apply to the other extremity of said network a potential controlled by an output signal of said memory means (18) for controlling the magnitude of the sensed heart signal applied to the input amplifier.

Although several levels of signal level attenuation are possible (and from which selection can be made), it has been found that two levels are sufficient in practice ("high" and "low"). Appropriate selection of one of these two levels can be accomplished simply by a single binary bit held in the program store.

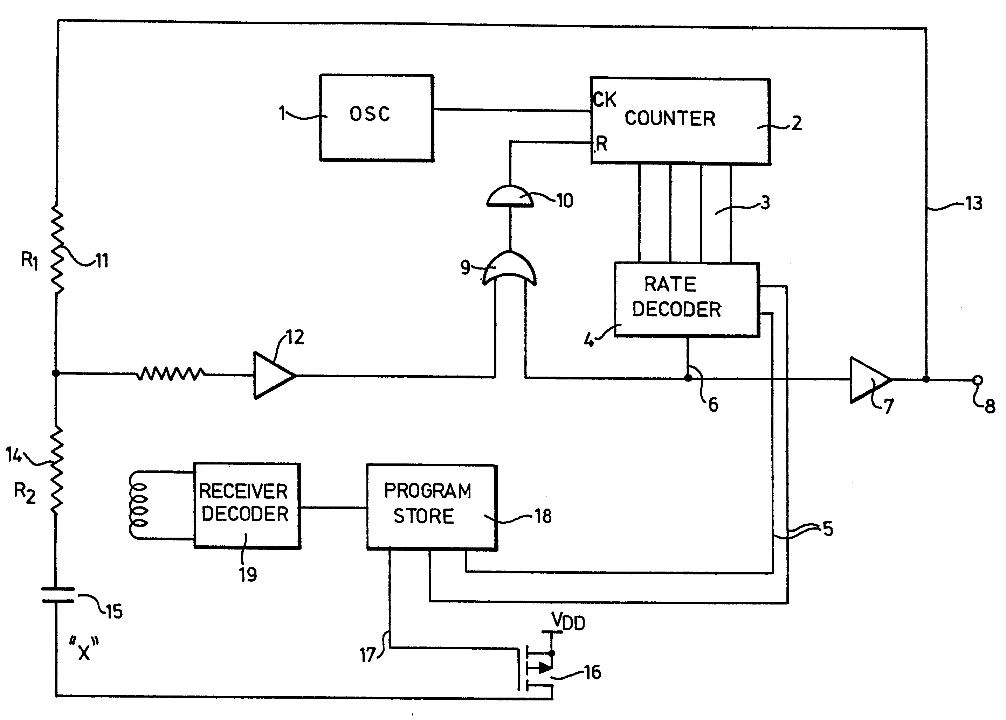

Brief description of the drawing

Preferred features of the invention are illustrated, by way of example, in the accompanying drawing which shows schematically an electrical circuit diagram of an implantable demand cardiac pacemaker according to the invention.

Best mode of making the invention

Referring to the drawing, the pacemaker comprises an oscillator 1 which clocks a ripple counter 2. Various outputs from the ripple counter are combined as is known in the art by means of logic gates (not shown) to provide four output lines 3. The oscillator frequency and combination of ripple counter outputs are selected so that the four output lines 3 provide, respectively, body stimulation pulses at four different frequencies (e.g. 50, 60, 70, 80 pulses per minute). The four output lines 3 are supplied to a rate decoder 4 provided with two input control lines 5. By employing binary logic circuitry, the logic levels on lines 5 can be employed to select uniquely one from four of the four lines 3 and transmit the selected pulse frequency on line 6. Line 6 is connected to an output amplifier 7 which in turn is connected to a connection 8 which leads to an active stimulating pulse electrode (not shown) disposed in or on the heart. Line 6 also provides one input to an OR gate 9.

The output of OR gate 9 is supplied to a delay unit 10 whose output is employed to reset counter 2. The delay generated by delay unit 10 sets the pulse width for each artificial stimulating pulse which is transmitted on output line 6.

The active electrode disposed in or on the heart feeds back the electrical signals detected at the heart (arising from an artificial stimulating pulse or a natural heart beat) to the pacemaker via a resistor 11 ($R_1$). The output of resistor $R_1$ is connected to an input amplifier 12 which in turn supplies a second input to OR gate 9. The feedback loop for such signals through the active electrode is illustrated by means of line 13.

Connected to input amplifier 12 is a second resistor 14 ($R_2$), which is connected via a capacitor 15 to the drain of a field effect transistor 16, the source of which is connected to $V_{DD}$. The gate of the transistor 16 is controlled by a control line 17 which, as with control lines 5, extends from a pacemaker program store 18. The program store 18 is arranged to hold, as a pacemaker program, a series of binary values for supply on the control lines 5 and 17.

A receiver/decoder 19 is arranged to receive and decode data signals transmitted from outside the patient's body to the implanted pacemaker, and to employ the decoded signals for changing a pacemaker program held in program store 18. For the purposes of illustration, the receiver/decoder 19 and store 18 have been depicted simply in functional box-form. The data signals may be transmitted to the receiver/decoder 19 by any suitable means, but preferably we employ data signals transmitted by tone burst modulation (pulse width modulation of a carrier frequency).

The pacemaker operates as follows. A program is entered and held in the program store 18 and the binary levels on control lines 5 determine which one of the four outputs 3 derived from counter 2 is passed by rate decoder 4 onto output line 6.

In the absence of natural heart beats, the counter 2 successively generates a series of artificial pulses at the selected rate and these are supplied to the heart via output amplifier 7. Each artificial pulse causes a reset of counter 2 via OR gate 9 and delay 10 to enable the count for the next artificial pulse to commence.

If a natural heart beat arises, an electrical signal is received by input amplifier 12 and counter 2 is reset (via OR gate 9 and delay 10) to inhibit any count in the process of being generated. Thus described, the pacemaker operates in a normal demand mode—issuing artificial pulses only when demanded by the heart. It is of no consequence if the reset initiated by a natural heart beat arrives just as an artificial pulse is generated, since the natural beat and the stimulating pulse will essentially coincide.

Should it be wished to vary the overall gain of the input amplifier, then this may be accomplished as follows. If a "1" is held in program store 18 for output on line 17 ("1"=$V_{DD}$), then the gate voltage of transistor 16 equals $V_{DD}$ and maintains the latter non-conducting: the point "X" between capacitor 15 and transistor 16 drain "floats". In such a circumstance $R_2$ has no influence on the magnitude of the input signal to input amplifier 12, as applied through resistor $R_1$.

If a "0" is held in the program store 18 for output on line 17, transistor 16 conducts and this causes point "X" to be at $V_{DD}$. The result of this is to cause $R_1$ and $R_2$ to act as a potential divider for the input signal, causing the input signal to the input amplifier to be attenuated.

In the normal circumstances, a "0" is held in the program store 18 and this causes attenuation of the input signal to amplifier 12. The gain of amplifier 12 and other parameters of the circuit are set such that the attenuation is acceptable and that the attenuated signal received is still of sufficient magnitude to carry out its necessary function of controlling the demand function of the pacemaker.

Should it be desired to increase the overall gain of the input amplifier (e.g. when there is an electrode displacement, or when the pacemaker batteries have suffered some loss of power after a relatively long period of use or when there is poor electrode placement, or the growth of tissue, all of which alter the signal "seen" by the pacemaker), then the program is changed to "1", to cause $R_2$ to float and remove attenuation of the input signal.

It will be appreciated that the circuit described above has been described in simple form, schematically, for the purposes of clarity. Also, though the amplifiers have been shown as single-input components it is of course possible to employ two input amplifiers (e.g. differential amplifiers).

## Claims

1. An implantable cardiac pacemaker having a pacemaker function programmably alterable from a remote source of pacemaker function programming signals comprising pulse generating means (1, 2, 3, 4) for periodically issuing stimulating pulses, an input amplifier (12) for sensing signals at the heart and for resetting said generating means in response to a sensed signal, and varying means including memory means (18) responsive to signals from said remote source for varying the signal provided by the input amplifier (12) for resetting said pulse generating means, characterised in that said varying means comprises a potential dividing network (11, 14) provided with an intermediate tap connected to the input of said input amplifier (12), means (13) connecting one extremity of said network to sense signals at the heart, and means (15, 16, 17) connected to apply to the other extremity of said network a potential controlled by an output signal of said memory means (18) for controlling the magnitude of the sensed heart signal applied to the input amplifier.

2. An implantable cardiac pacemaker according to claim 1, characterised in that said means connected to apply a potential comprise a voltage source ($V_{DD}$), a capacitor (15) connected on one side to said other extremity of said network, and a switch means (16) between said source ($V_{DD}$) and the other side of said capacitor (X), said switch means (16) operating in response to the output signal of said memory means.

3. An implantable cardiac pacemaker according to claim 2, characterised in that said switch means (16) comprises a field effect transistor whose gate electrode receives (17) said output signal of said memory means.

## Patentansprüche

1. Implantierbarer Herzschrittmacher mit einer Schrittmacherfunktion, welche programmierbar veränderbar ist von einer getrennten Programmiersignalquelle, die enthält: Impulserzeugungsmittel (1, 2, 3, 4) zur periodischen Ausgabe von Stimulationsimpulsen, einen Eingangsverstärker (12) zum Erfassen von Herzsignalen und zum Zurücksetzen der Erzeugungsmittel in Abhängigkeit von einem aufgenommenen Signal sowie Veränderungsmittel, die Speichermittel (18) umfassen, welche auf Signale von der getrennten Programmiersignalquelle zur Veränderung des Signals, das welches von dem Verstärker (12) geliefert wird, zum Zurücksetzen der Impulserzeugungsmittel, dadurch gekennzeichnet, daß die Veränderungsmittel ein Potentialteilernetzwerk (11, 14) aufweisen, welches mit einer Anzapfung versehen ist, die mit dem Eingang des Eingangsverstärkers (12) verbunden ist, sowie Mittel (13), welche ein Ende des Netzwerks zur Signalaufnahme vom Herzen verbinden und Mittel (15, 16, 17), die mit dem anderen Ende des Netzwerks verbunden sind um ein Potential anzulegen, welches von einem Ausgangssignal der Speichermittel (18) beeinflußt wird, um die Größe der Amplitude der aufgenommenen Herzsignale, die dem Eingang des Verstärkers zugeführt werden, zu steuern.

2. Implantierbarer Herzschrittmacher nach Anspruch 1, dadurch gekennzeichnet, daß die Mittel zum Erzeugen eines Potentials eine Spannungsquelle ($V_{DD}$), einen Kondensator (15), welcher mit dem einen Ende des Netzwerks verbunden ist, und Schaltmittel (16) zwischen der Quelle ($V_{DD}$) und dem anderen Ende des Kondensators (X) aufweisen, wobei die Schaltmittel (16) in Abhängigkeit vom Ausgangssignal der Speichermittel arbeiten.

3. Implantierbarer Herzschrittmacher nach Anspruch 2, dadurch gekennzeichnet, daß die Schaltmittel (16) einen Feldeffekttransistor enthalten, dessen Gate-Elektrode das Ausgangssignal der Speichermittel zugeführt erhält (17).

## Revendications

1. Stimulateur cardiaque implantable possédant une fonction de stimulateur qui peut être modifiée par programmation à partir d'une source éloignée de signaux de programmation de fonction de stimulateur, comprenant un moyen (1, 2, 3, 4) générateur d'impulsions qui délivre périodiquement des impulsions de stimulation, un amplificateur d'entrée (12) qui détecte des signaux au niveau du coeur et qui repositionne ledit moyen générateur en réponse à un signal détecté, et un moyen de variation comportant un moyen de mémorisation (18) qui répond à des signaux en provenance de ladite source éloignée en faisant varier le signal fourni par l'amplificateur d'entrée (12) pour repositionner ledit moyen générateur d'impulsions, caractérisé en ce que ledit moyen de variation comprend un réseau diviseur de tension (11, 14) doté d'une prise intermédiaire connectée à l'entrée dudit amplificateur d'entrée (12), un moyen (13) qui connecte une première extrémité dudit réseau afin de détecter des signaux au niveau du coeur, et un moyen (15, 16, 17) connecté de façon à appliquer à l'autre extrémité dudit réseau un potentiel commandé par un signal de sortie dudit moyen de mémorisation (18) de façon à commander l'amplitude du signal cardiaque détecté qui est appliqué à l'amplificateur d'entrée.

2. Stimulateur cardiaque implantable selon la revendication 1, caractérisé en ce que ledit moyen connecté de façon à appliquer un potentiel comprend une source de tension ($V_{DD}$), un condensateur (15) connecté par un premier de ses côtés à ladite autre extrémité dudit réseau, et un moyen de commutation (16) disposé entre ladite source ($V_{DD}$) et l'autre côté dudit condensateur (X), ledit moyen de commutation (16) s'activant en réponse au signal de sortie dudit moyen de mémorisation.

3. Stimulateur cardiaque implantable selon la revendication 2, caractérisé en ce que ledit moyen de commutation (16) comprend un transistor à effet de champ dont l'électrode de grille reçoit (17) ledit signal de sortie dudit moyen de mémorisation.

OSC

COUNTER
CK
R

RATE
DECODER

PROGRAM
STORE

RECEIVER
DECODER

$V_{DD}$

$R_1$

$R_2$

"X"

1